# FASCICULE DE BREVET EUROPEEN

(11) **EP 1 408 921 B1**
(45) Date de publication et mention de la délivrance du brevet: **22.08.2007**
(21) Numéro de dépôt: 02782462.2
(22) Date de dépôt: 03.07.2002
(51) Int. Cl.: A61K 8/21, A61K 8/23, A61K 8/67, A61Q 11/00

(54) **COMPOSITION POUR L'HYGIENE BUCCO-DENTAIRE COMPRENANT UN VECTEUR D'IONS FLUORURE ET UN AGENT ANTIOXYDANT**
MUNDPFLEGEMITTEL ENTHALTEND EINEN FLUOR-ION-VEKTOR UND EIN ANTIOXIDATIONSMITTEL
COMPOSITION FOR ORAL HYGIENE COMPRISING A FLUORIDE ION VECTOR AND AN ANTIOXIDANT

(30) Priorité: 04.07.2001 FR 0108852
(43) Date de publication de la demande: 21.04.2004
(73) Titulaire: The Procter and Gamble Company, Cincinnati, Ohio 45202 (US)
(72) Inventeur: CAZOR, Jean-Louis, F-75004 Paris (FR); LHUISSET, François, F-91230 Montgeron (FR); ROBINEAU, Pascale, F-91120 Palaiseau (FR)
(74) Mandataire: Michelet, Alain
(86) Numéro de dépôt international: PCT/FR2002/002313
(87) Numéro de publication internationale: WO 2003/003995

(56) Documents cités:
- GB-A- 1 018 654
- GB-A- 1 525 254
- US-A- 4 069 312
- US-A- 5 174 989
- DATABASE WPI Section Ch, Week 199933 Derwent Publications Ltd., London, GB; Class B05, AN 1999-386214 XP002196950 & CN 1 213 534 A (CHEN C), 14 avril 1999 (1999-04-14)
- DATABASE WPI Section Ch, Week 199730 Derwent Publications Ltd., London, GB; Class B05, AN 1997-331186 XP002196951 & RU 2 070 030 C (OLIFEN STOCK CO), 10 décembre 1996 (1996-12-10)

## Description

L'invention se rapporte aux compositions pour l'hygiène bucco-dentaire ainsi qu'à leurs utilisations.
Les pathologies bucco-dentaires sont fréquentes chez les sujets diabétiques. De plus, chez ces sujets, une infection de la sphère buccale peut déséquilibrer le diabète et entraîner la dissémination des germes dans l'organisme. Plusieurs études ont ainsi montré qu'une amélioration de l'état gingival pouvait faciliter le contrôle de la glycémie (Mealey, B. (2000) Diabetes and periodontal diseases, J. Periodontol. 71, 664-678).

Il est également connu que le diabète multiplie par un facteur d'environ trois le risque de développer une maladie parodontale (Emrich L.C. et al (1991) Periodontal disease in non-insulin dependent diabetes mellitus, J. Periodontol. 62, 123-130). D'autres études ont montré que la fréquence des caries et la prévalence de stomatites de diverses origines sont augmentées chez les sujets diabétiques (Twetman S. et al, (1992) Two-year longitudinal observations of salivary status and dental caries in children with insulino-dependent diabetes mellitus, Pediatr. Dent. 14, 184-188).

La fréquence relativement élevée des pathologies bucco-dentaires mentionnées ci-dessus chez les sujets diabétiques est notamment liée aux dysfonctionnements suivants. D'une part, l'hyperglycémie provoque la glycosylation et l'oxydation non enzymatiques de lipides et de protéines, ainsi que l'activation de récepteurs RAGE (« Receptor for Advanced Glycation End products »), présents sur la surface membranaire de nombreux tissus. Ces réactions biochimiques entraînent notamment une augmentation du stress oxydatif des cellules et, finalement, le développement d'un environnement pro-inflammatoire, favorable à la formation de lésions et conduisant à la défaillance des mécanismes réparateurs.
Les radicaux libres de l'oxygène sont en effet responsables de l'altération de l'ADN et des molécules constitutives telles que le collagène et l'élastine, de la destruction des mucopolysaccharides, de changements délétères dans la composition des membranes et de lésions vasculaires.
Pour combattre les effets néfastes des radicaux libres de l'oxygène, il a été proposé d'administrer aux sujets diabétiques des composés à activité antioxydante. Il a été ainsi montré, par exemple, qu'une supplémentation orale avec un antioxydant comme la Vitamine E réduisait chez les sujets diabétiques les risques de rétinopathie et de néphropathie, complications liées au dysfonctionnement vasculaire induit par le diabète (Bursell S.E. et al, (1999) High-dose Vitamin E supplementation normalizes retinal blood flow and creatinine clearance in patients with type I diabetes, Diabetes Care 22, 1245-1251).

L'activité de la Vitamine E au niveau bucco-dentaire a été cependant peu explorée. *In vitro,* elle est capable de réduire le stress oxydatif subi par des cellules d'épithélium buccal humain en présence d'eau oxygénée (Royack G.A. et al, (2000) Response of human oral epithelial cells to oxidative damage ans the effect of vitamin E, Oral Oncology 36, 37-41).
Appliquée localement sur des zones buccales touchées par une mycose - affection souvent provoquée par *Candida albicans* et fréquente chez les sujets diabétiques - , la Vitamine E entraîne la résolution rapide des lésions (Wadleigh R.G. et al, (1992) Vitamin E in the treatment of chemotherapy-induced mucositis, Am. J. Med. 92, 481-484).

Les effets de la Vitamine E sur l'état gingival en application topique ont par ailleurs été décrits comme inexistants (Lange D.E. et al, (1975) Management of gingival inflammation with active ingredients in toothpaste, Dtsch. Zahnärztl. Z. 30, 382-384 ; Cohen R.E. et al, (1991) Effect of vitamin E gel, placebo gel and chlorhexidine on periodontal disease, Clin. Prev. Dent. 13, 20-24).

La fréquence des pathologies bucco-dentaires chez les sujets diabétiques est liée d'autre part à la modification du milieu buccal, liée à la maladie diabétique et à son traitement, ainsi qu'à la défaillance de la réponse immunitaire qui entraînent un déséquilibre de la flore bactérienne, souvent au profit de souches pathogènes, et le développement de microorganismes opportunistes.

Il a ainsi été montré que des bactéries pathogènes pour le tissu parodontal, telles *Porphyromonas gingivalis, Capnocytophaga ochracea, Fusobacterium nucleatum, Prevotella intermedia,* sont trouvées plus fréquemment dans la flore buccale des sujets diabétiques (Sbordone L. et al, (1998) Periodontal status and subgingival microbiota of insulin-dependent juvenil diabetics : A 3-year longitudinal study, J. Periodontol. 69, 120-128).
La réduction du flux salivaire favorise également la survenue de caries. La prévalence d'infections liées à *Helicobacter pylori,* bactérie responsable d'ulcères gastro-intestinaux qui est présente dans la plaque dentaire et se transmet oralement, est également plus élevée chez les patients diabétiques (Quadri R. et al, (2000) Helicobacter pylori infection in type 2 diabetic patients, Nutr. Metab. Cardiovasc. Dis. 10, 263-266), pour lesquels l'éradication de la bactérie est en outre plus difficile que pour les sujets normaux (Gasbarrini A. et al, (1999) Insulin-dependent diabetes mellitus affects eradication rate of Helicobacter pylori infection, Eur. J. Gastroenterol. Hepatol. 11, 713-716).

De nombreuses compositions à usage bucco-dentaire ont déjà été décrites. Par exemple, les documents WO 0028977 et WO 9900106 décrivent des compositions, contenant au minimum deux antioxydants à titre de principes actifs, destinées à l'usage des fumeurs ou à un usage général.
Ainsi, dans le document WO 0028977, il est décrit une composition de gel ou de pâte dentaire comprenant comme ingrédients actifs du glutathion « réduit » (GSH) et une source de sélénium, associés ou non à d'autres constituants, pour la prévention et le traitement des symptômes gingivaux.
Le document WO 9900106 décrit des compositions pour l'hygiène bucco-dentaire à l'usage des fumeurs comprenant du glutathion « réduit » et une source de sélénium, ainsi que d'autres constituants.
Toutefois, l'incorporation de GSH en quantité significative dans une pâte dentifrice ou un gel est difficile à mettre en oeuvre sur le plan industriel, aussi bien en raison de l'instabilité de ce composé à température ambiante qu'en raison de son coût.

Il n'existe pas par ailleurs à l'heure actuelle de composition pour l'hygiène bucco-dentaire plus particulièrement capable de prévenir et/ou combattre les pathologies bucco-dentaires des sujets diabétiques.

L'invention vise à remédier à ces inconvénients, en proposant une composition pour l'hygiène bucco-dentaire adaptée aux sujets diabétiques et présentant une activité biologique en situation normale, avec un produit stable et simple à formuler.

Un premier objet de l'invention concerne donc une composition pour l'hygiène bucco-dentaire, comprenant à titre de principes actifs au moins un vecteur d'ions fluorure et au moins un agent antioxydant stable, ledit agent antioxydant n'étant pas le glutathion.

Un deuxième objet de l'invention concerne l'utilisation d'un vecteur d'ions fluorures et d'un agent antioxydant pour la préparation d'une composition pour l'hygiène bucco-dentaire destinée à combattre et/ou prévenir les pathologies bucco-dentaires chez les sujets diabétiques.

Selon un premier aspect, l'invention concerne une composition pour l'hygiène bucco-dentaire, destiné à combattre et/ou prévenir les pathologies bucco-dentaires chez les sujets diabétiques, comprenant à titre de principes actifs au moins un vecteur d'ions fluorures et au moins un agent antioxydant stable, ledit agent antioxydant n'étant pas le glutathion.

Selon l'invention, la composition comprend en tant que premier principe actif au moins un agent antioxydant, à l'exception du glutathion.
Selon l'invention, on entend par le terme « agent antioxydant » un composé naturel ou non qui est capable de réduire les dommages causés par les radicaux libres de l'oxygène.
Les agents antioxydants diminuent le stress oxydatif des cellules et exercent un rôle protecteur des systèmes biologiques vis-à-vis des pathologies associées aux radicaux libres de l'oxygène.
Les antioxydants jouent également un rôle dans la protection contre la contamination par *Hélicobacter pylori,* la prévalence de l'infection semblant liée à une faiblesse des défenses antioxydantes.

Des agents antioxydants utilisables selon l'invention sont notamment :
- l'alpha-tocophérol et ses dérivés, notamment la Vitamine E, et les sels de tocophéryl ;
- le coenzyme Q10 et ses dérivés, particulièrement l'ubiquinol 10 ;
- le vanadium et les sels de vanadium ;
- la Vitamine C et les sels d'acide ascorbique ;
- le pyruvate ;
- l'acide nicotinique ;
- la Vitamine A, le rétinol, l'alpha-carotène, le beta-carotène, le lycopène, la lutéine, la zéaxanthine et autres caroténoïdes ;
- la thiamine, la riboflavine, la pyridoxine et les Vitamines B1, B2 et B6 ;
- la mélatonine ;
- les flavonoïdes ;
- l'acide lipoïque ;
- les acides aminés soufrés tels que la méthionine, la cystéine et la N-acétylcystéine, et la taurine.

Des agents antioxydants préférés selon l'invention sont notamment l'alpha-tocophérol et ses dérivés, notamment la Vitamine E, éventuellement sous forme de sel, par exemple d'acétate.

Les compositions selon l'invention comprennent des agents antioxydants en une quantité telle que la teneur totale en agent antioxydant soit supérieure à 0,0001 % en poids, de préférence comprise entre 0,0005 et 2% en poids par rapport au poids total de ladite composition.

Selon l'invention, la composition comprend en tant que second principe actif au moins un vecteur d'ions fluorures.

Selon l'invention, on entend par le terme « vecteur d'ions fluorure » un composé fluoré, minéral ou organique, qui autorise la libération d'ions fluorure dans un milieu aqueux, tel que la salive.

Les ions fluorures sont connus pour augmenter la résistance des dents aux acides produits à partir des sucres présents dans la cavité buccale et pour prévenir le risque de carie dentaire. A teneur élevée, ils présentent également une activité antibactérienne sur les bactéries de la plaque dentaire et sur *Helicobacter pylori.*

Des vecteurs d'ions fluorure utilisables selon l'invention sont notamment :
- les fluorures d'amine, le fluorure d'ammonium, les fluorures de métaux alcalins, par exemple de sodium et de potassium, les fluorures alcalino-terreux, par exemple de calcium, les fluorures de titane, de zinc, d'étain, d'aluminium, ainsi que les dérivés de ces fluorures ;
- les fluorophosphates de métaux alcalins, par exemple de potassium ou de sodium, notamment le monofluorophosphate de sodium, les fluorophosphates de métaux alcalino-terreux, notamment de calcium, les fluorophosphates d'aluminium, notamment le monofluorophosphate d'aluminium, le difluorophosphate d'aluminium ;
- le fluorozirconate de sodium ;
- les fluorosilicates, tels que les fluorosilicates de sodium comme l'hexafluorosilicate de sodium.
   Des vecteurs d'ions fluorures préférés sont notamment le fluorure de sodium, le monofluorophosphate de sodium ou un mélange de fluorure de sodium et de monofluorophosphate de sodium.
   La composition selon l'invention comprend des vecteurs d'ions fluorure en une quantité telle que sa teneur en ions fluorure soit supérieure à 0,0001% en poids, de préférence comprise entre 0,001 et 2% en poids par rapport au poids total de la composition.
   Selon une forme d'exécution de l'invention, la composition de l'invention comprend un premier et un second agents antioxydants et au moins un vecteur d'ions fluorures.
   Selon cette forme d'exécution, le premier agent antioxydant est tel que défini dans ce qui précède et le second agent antioxydant est un produit sélénié.
   Des produits séléniés utilisables sont les composés sous forme de sel, notamment un sélénate, par exemple le sélénate de sodium; un sélénite, par exemple le sélénite de sodium; un composé séléno-organique, par exemple un composé avec un acide aminé tel que la sélénométhionine, la sélénocystéine.
   Les agents antioxydants agissant souvent en synergie, l'association de deux ou plus agents antioxydants présente donc un intérêt supplémentaire.
   Des associations préférées d'agents antioxydants sont notamment un mélange d'alpha-tocophérol ou de ses dérivés et un sélénate ou sélénite de sodium.
   Il va de soi que dans le cas où la composition de l'invention ne comprend qu'un seul agent antioxydant, cet agent antioxydant peut être choisi parmi les composés séléniés décrits ci-dessus.
   Les principes actifs décrits dans ce qui précède, ainsi que leurs procédés de préparation, sont tous connus ou disponibles commercialement.
   Les compositions de l'invention peuvent se présenter sous toute forme adaptée pour une application buccale locale. Une telle forme pour application buccale peut consister notamment en un dentifrice, un spray, une gomme à mâcher, une pastille à sucer, un gel dentaire pour application topique, un implant buccal tel qu'un patch buccal, notamment un patch muco-adhésif, un bain de bouche.
   Les compositions sous forme de dentifrice peuvent se présenter sous forme pâteuse, liquide, de gel, de poudre ou de mousse.
   Ces formes en elles-mêmes ainsi que leur préparation sont bien connues de l'homme du métier. Outre les vecteurs d'ions fluorure et les agents antioxydants, les formes pour application buccale mentionnées ci-dessus peuvent comprendre des excipients ou ingrédients conventionnels pour chacune de ces formes.
   Par exemple, les formes pour application buccale peuvent contenir des tensio-actifs anioniques, amphotères, zwittérioniques, cationiques ou non-ioniques. Elles peuvent encore comprendre des agents épaississants, des agents de cohésion, des agents édulcorants, humectants ou rafraîchissants, des agents conservateurs, des colorants, des agents blanchissants, des agents aromatisants ou de sapidité, des huiles essentielles de plante, des agents plastifiants, des agents peptisants, des agents anti-tartre, des agents inhibiteurs de la production de composés volatiles soufrés tels les sels et complexes de zinc, des cicatrisants, des agents anti-saignements, des agents de polissage, des agents anti-plaque dentaire tels que la chlorhexidine, l'héxétidine, le chlorure de cétylpyridinium, le triclosan et/ou des enzymes comme la dextranase, la mutanase, les lysozymes, la lactoferrine ou les peroxydases.
   Selon un autre aspect, l'invention concerne l'utilisation d'au moins un agent antioxydant et d'au moins un vecteur d'ions fluorures pour la préparation d'une composition pour l'hygiène bucco-dentaire destinée à prévenir et/ou combattre les pathologies bucco-dentaires chez les sujets diabétiques.
   Selon l'invention, les agents antioxydants et les vecteurs d'ions fluorures, ainsi que leurs concentrations respectives, sont tels que décrits dans ce qui précède.
   Des caractéristiques et avantages supplémentaires de l'invention apparaîtront dans les exemples qui suivent.
   En vue d'étudier l'efficacité d'un produit d'hygiène bucco-dentaire de l'invention destiné aux sujets diabétiques, des compositions sous forme de dentifrice conformes à l'invention comprenant les composés suivants (exprimés en pourcentage en poids par rapport au poids total de la composition) sont préparées :

### Composition 1

- fluorure de sodium 0,33
- monofluorophosphate de sodium 0,76
- DL-alpha-tocopherol (vitamine E) 0,5
- silices 21,00
- sorbitol 25,00
- benzoate de sodium 4,00
- phosphates 0,3
- carraghénate 1,00
- dioxyde de titane 1,00
- tensio-actif 2,00
- conservateur 0,1
- arôme 1,0
- eau purifiée q.s.p.

### Composition 2

- fluorure de sodium 0,55
- DL-alpha-tocopherol (vitamine E) 0,5
- silices 20,00
- glycérol 32,00
- carboxyméthylcellulose 1,00
- dioxyde de titane 1,00
- tensio-actif 1,00
- conservateur 0,30
- arôme 0,2
- eau purifiée q.s.p.

Les résultats suivants ont été obtenus.

### Exemple 1.

### Etude de la présence prolongée de la Vitamine E dans la salive après brossage.

La disponibilité et la rémanence de la Vitamine E dans la salive après brossage est étudiée chez neuf sujets, qui ont utilisé la composition 1 deux fois par jour pendant deux jours, pour des brossages d'une durée de trois minutes. La Vitamine E est indétectable dans la salive avant brossage, elle atteint une concentration moyenne de 259 µg / g à la fin du brossage et est encore présente, à la concentration moyenne de 3 µg / g, trois heures après le brossage en utilisant la composition de l'invention.

### Exemple 2.

### Etude de l'effet antibactérien sur les bactéries de la plaque dentaire.

A titre comparatif, on prépare un dentifrice non conforme à l'invention, comprenant les mêmes composés que la composition 1 ci-dessus, à l'exception du fluorure de sodium, du monofluorophosphate de sodium et de la Vitamine E (placebo).
Des milieux de culture sont ensemencés avec des souches bactériennes représentatives de souches présentes dans la plaque dentaire, dans des conditions favorables à leur développement. Les milieux contenant les bactéries sont mis ensuite en contact avec soit la composition 1, soit la composition placebo, après dilution des compositions au 1/2, au 1/4 et au 1/8.

Les résultats obtenus après cinq minutes de contact sont indiqués dans le tableau I ci-après.

**Tableau I.**

| Dilution Souche | 1/2 | | 1/4 | | 1/8 | |
|---|---|---|---|---|---|---|
| | Comp. 1 | Placebo | Comp. 1 | Placebo | Comp. 1 | Placebo |
| *Streptococcus mutans* | 4,7 (6,8) | 19 (11) | 5,9 (0,6) | 27 (1,5) | 8,0 (0,4) | 25 (1,9) |
| *Streptococcus salivarius* | 4,7 (1,2) | 23 (3,5) | 8,1 (0,4) | 20 (3) | 12 (3,4) | 23 (2,5) |
| *Lactobacillus Casei* | 5,9 (0,2) | 18 (5) | 6,5 (2,4) | 19 (2,6) | 13 (1,2) | 22 (4,5) |
| *Actinomyces Odontolyticus* | 5,1 (2,3) | 20 (1,5) | 7,2 (2,5) | 22 (2,5) | 20 (1) | 29 (0,8) |
| *Actinobacillus Actinomycetemcomitans* | 7,3(0,4) | 9,0(1,5) | 7,2(1,0) | 9,1 (1,1) | 17 (1,0) | 17 (2,0) |
| *Eikenella Corrodens* | 8,2 (1,5) | 11 (1,4) | 6,9(1,1) | 11(1,9) | 9(0,9) | 17 (0,6) |
| *Capnocytophaga ochracea* | 3,8 (1,5) | 8,0 (0,4) | 6,2 (1,1) | 10 (0,3) | 12 (0,8) | 19 (0,8) |
| *Porphyromonas gingivalis* | 0,9 (0,1) | 2,0 (1,4) | 1,0 (0,1) | 2,2 (2,5) | 1,4 (1,1) | 2,3 (1,1) |
| *Prevotella intermedia* | 0,9 (0,5) | 2,2 (1,1) | 0,9 (0,4) | 2,2 (3,5) | 1,5 (5,4) | 1,9 (2,1) |
| *Bacteroides Loeschii* | 10 (1,2) | 26 (9) | 9,8 (0,5) | 25 (20,4) | 22 (2,5) | 25 (0,6) |
| *Prevotella Melaninogenicus* | 5,0 (0,7) | 18 (3,6) | 6,7 (16,0) | 21 (3,0) | 13,5 (4,5) | 22 (1,5) |
| *Fusobacterium nucleatum* | 2,1 (0,1) | 16 (7,6) | 2,4 (6,8) | 15 (3,6) | 14 (3,6) | 16 (1,5) |
| *Campylobacter Rectus* | 10 (1,5) | 16 (5,0) | 9,0 (0,3) | 15 (0,3) | 17 (0,3) | 18 (10,0) |
| *Peptostreptococcus micros* | 4,0 (0,8) | 14 (1,5) | 8,3(0,3) | 19 (7,6) | 19 (1,5) | 22 (3,0) |

Les valeurs indiquées dans le tableau I représentent le nombre d'Unités formant des Colonies (UFC) à multiplier par 10⁵. Chacune de ces valeurs représente une moyenne de trois tests. La déviation standard est indiquée entre parenthèses.

Ces résultats montrent que la composition de l'invention réduit le nombre d'UFC de la plupart de ces souches, après un temps de contact de cinq minutes seulement.

### Exemple 3.

### Effet antibactérien sur Helicobacter pylori.

A titre comparatif, un dentifrice non conforme à l'invention (placebo), comprenant les composés indiquées pour la composition 1 ci-dessus, à l'exception du fluorure de sodium, du monofluorophosphate de sodium et de la Vitamine E (placebo), est préparé.
Un milieu de culture est ensemencé avec une souche d'Helicobacter pylori, dans des conditions favorables à son développement. Les milieux contenant les bactéries sont mis ensuite en contact avec soit la composition 1 de l'invention, soit la composition placebo, après dilution des compositions au 1/2, au 1/4, au 1/8 et au 1/16.

Les résultats obtenus après cinq minutes, une heure et vingt-quatre heures de contact sont indiqués dans les tableaux II (placebo) et III (composition 1) ci-après.

**Tableau II**

| Dilution Temps de contact | 1/2 | 1/4 | 1/8 | 1/16 |
|---|---|---|---|---|
| 5 minutes | 10(2) | 20 (2,1) | 35 (1,7) | 35 (3,4) |
| 1 heure | 9,5 (1,2) | 17 (1,9) | 35 (0,8) | 44 (2,8) |
| 24 heures | 9,2 (0,2) | 9,5 (0,3) | 42 (0,5) | 48 (20) |

**Tableau III**

| Dilution Temps de contact | 1/2 | 1/4 | 1/8 | 1/16 |
|---|---|---|---|---|
| 5 minutes | 5 (0,2) | 8 (0,5) | 11 (0,8) | 25 (2,5) |
| 1 heure | 2,9 (0,1) | 5,2 (0,3) | 10 (1,9) | 19 (5,6) |
| 24 heures | 1,2 (0,3) | 1,4 (0,4) | 10(0,9) | 40 (4,2) |

Les valeurs indiquées dans les tableaux II et III représentent le nombre d'Unités formant des Colonies (UFC) à multiplier par 10⁵. Chacune de ces valeurs représente une moyenne de trois tests. La déviation standard est indiquée entre parenthèses.

Ces résultats montrent que la composition de l'invention réduit fortement le nombre d'UFC d'*Helicobacter pylori,* après un temps de contact de cinq minutes seulement.

### Exemple 4.

### Amélioration de l'état gingival.

A titre comparatif, deux compositions non conformes à l'invention sont préparées :
- l'une comprenant les composés indiqués dans la composition 1 ci-dessus, à l'exception du fluorure de sodium, du monofluorophosphate de sodium et de la Vitamine E (placebo),
- l'autre comprenant les composés indiqués dans la composition 1 ci-dessus, à l'exception de la Vitamine E (témoin fluoré).

L'efficacité de la composition 1 est mise en évidence dans une étude clinique réalisée sur vingt-quatre volontaires. Pendant une première phase de quatorze jours, tous les sujets se brossent les dents avec le placebo. Ensuite, ils utilisent deux fois par jour pendant vingt et un jours le dentifrice qui leur est attribué parmi le dentifrice de l'invention (composition 1) et les deux autres dentifrices non conformes à l'invention mentionnés ci-dessus, dans les conditions suivantes : une partie de la dentition (dents 32 à 37) est protégée de l'action mécanique du brossage par un cache, dans lequel deux millilitres du dentifrice sont introduits, tandis que les autres dents sont brossées normalement pendant deux minutes.
Chaque sujet reçoit successivement les trois dentifrices pendant vingt et un jours, selon un ordre déterminé par randomisation, avec une période de quatorze jours sous placebo entre chaque traitement.
L'état d'inflammation de la gencive dans la partie de la bouche non brossée pendant le traitement est évalué, sur des répliques, par la mesure de l'épaisseur gingivale au début de la gencive attachée à partir de la dent, selon la méthode tridimensionnelle décrite par Jovanovski V. et al, (2000) Analysis of the morphology of oral structures from 3-D co-ordinate data, Monogr. Oral Sci. 17, 73-129.

Les résultats obtenus après vingt et un jours de traitement sont indiqués dans le tableau IV ci-après.

**Tableau IV**

| Traitement | Composition 1 | Témoin fluoré | Placebo |
|---|---|---|---|
| Epaisseur de la gencive (différence en µm) | -82,6 (68,6) ** | -19,8 (76,4) | -15,4 (111,7) |

Les valeurs indiquées dans le tableau IV représentent la différence d'épaisseur (en µm) de la gencive entre le début et la fin du traitement. Chacune de ces valeurs représente une moyenne sur 20 à 22 sujets. La déviation standard est indiquée entre parenthèses.

L'analyse statistique (Duncan's Multiple Range) met en évidence une diminution de l'épaisseur de la gencive plus importante après utilisation de la composition 1 selon l'invention (**, P<0.01) qu'après utilisation du témoin fluoré ou du placebo. Compte tenu des dimensions de la gencive au point de mesure, cet effet représente une réduction de plus de 10% de l'épaisseur gingivale.

Ces résultats montrent que la composition de l'invention, de part la présence d'un antioxydant stable dans la formulation et en quantité suffisante, améliore significativement l'état de la gencive.

D'autres exemples de compositions sous forme de gomme à mâcher ou de bain de bouche sont donnés ci-après.

### Gomme à mâcher

La composition suivante (en pourcentage en poids par rapport au poids total de la composition) est utilisée pour préparer une gomme à mâcher conforme à l'invention :
- fluorure de sodium 0,005
- DL-alpha-tocopherol (vitamine E) 0,005
- taurine 2,0
- gomme 32,0
- xylitol 35,0
- glycérol 2,0
- arôme 4,0
- sorbitol q.s.p.

### Bain de bouche

La composition suivante (en pourcentage en poids par rapport au poids total de la composition) est utilisée pour préparer un bain de bouche conforme à l'invention :
- fluorure de sodium 0,01
- sélénate de sodium 0,0005
- glycerol 5,00
- conservateur 0,20
- xylitol 7,00
- tensio-actif 1,00
- colorant 0,20
- arôme 0,05
- eau purifiée q.s.p.

## Revendications

1. Composition pour l'hygiène bucco-dentaire, destiné à combattre et/ou prévenir les pathologies parondontales du sujet diabétique, contenant à titre de principes actifs de la Vitamine E et au moins un vecteur d'ions fluorure, à une teneur en ions fluorure supérieure à 0,0001 % en poids, de préférence comprise entre 0,001 et 2% en poids par rapport au poids total de la composition.

2. Composition selon la revendication 1, **caractérisée en ce que** la Vitamine E est présente sous forme de sel, par exemple d'acétate.

3. Composition selon la revendication 1 ou 2, **caractérisée en ce que** le vecteur d'ions fluorures est notamment le fluorure d'amine, le fluorure d'ammonium, les fluorures de métaux alcalins, par exemple de sodium et de potassium, les fluorures alcalino-terreux, par exemple de calcium, les fluorures de titane, de zinc, d'étain, d'aluminium, ainsi que les dérivés de ces fluorures ; les fluorophosphates d'aluminium, les fluorophosphates de métaux alcalins, notamment de sodium et de potassium, par exemple le monofluorophosphate de sodium, les fluorophosphates alcalino-terreux, par exemple de calcium, les fluorophosphates d'aluminium, notamment le monofluorophosphate d'aluminium; le difluorophosphate d'aluminium ; la fluorozirconate de sodium ; les fluorosilicates, tels que les fluorosilicates de sodium comme l'hexafluorosilicate de sodium.

4. Composition selon la revendication 3, **caractérisée en ce que** le vecteur d'ions fluorures est le fluorure de sodium, le monofluorophosphate de sodium ou un mélange de ceux-ci.

5. Composition selon l'une quelconque des revendications 1 à 4, **caractérisée en ce qu'**elle comprend à titre de principes actifs un second agent antioxydant.

6. Composition selon la revendication 5, **caractérisée en ce que** le second agent antioxydant est un produit sélénié.

7. Composition selon la revendication 6, **caractérisée en ce que** le produit sélénié est notamment un sélénate, par exemple le sélénate de sodium; un sélénite, par exemple le sélénite de sodium; un composé séléno-organique, par exemple un composé avec un acide aminé tel que la sélénométhionine, la sélénocystéine.

8. Composition selon l'une quelconque des revendications 1 à 7, **caractérisée en ce que** la teneur totale en agents antioxydants est supérieure à 0,0001% en poids, de préférence comprise entre 0,0005 et 2% en poids par rapport au poids total de ladite composition.

9. Composition selon l'une quelconque des revendications 1 à 8, utilisable sous forme de dentifrice, **caractérisée en ce qu'**elle comprend les composés suivants, exprimés en pourcentage en poids par rapport au poids total de la composition :
- fluorure de sodium 0,33
- monofluorophosphate de sodium 0,76
- DL-alpha-tocopherol (vitamine E) 0,5
- silices 21,00
- sorbitol 25,00
- benzoate de sodium 4,00
- phosphates 0,3
- carraghénate 1,00
- dioxyde de titane 1,00
- tensio-actif 2,00
- conservateur 0,1
- arôme 1,0
- eau purifiée q.s.p.

10. Composition selon l'une quelconque des revendications 1 à 8, utilisable sous forme de dentifrice, **caractérisée en ce qu'**elle comprend les composés suivants, exprimés en pourcentage en poids par rapport au poids total de la composition :
- fluorure de sodium 0,55
- DL-alpha-tocopherol (vitamine E) 0,5
- silices 20,00
- glycérol 32,00
- carboxyméthylcellulose 1,00
- dioxyde de titane 1,00
- tensio-actif 1,00
- conservateur 0,30
- arôme 0,2
- eau purifiée q.s.p.

11. Composition selon l'une quelconque des revendications 1 à 10, **caractérisée en ce qu'**elle est formulée sous forme de dentifrice; de spray pour application buccale, de gomme à mâcher, de pastille à sucer, de patch buccal, de bain de bouche.

12. Utilisation d'au moins un agent antioxydant et d'au moins un vecteur d'ions fluorures pour la préparation d'une composition pour l'hygiène bucco-dentaire destinée à prévenir et/ou combattre les pathologies parodontales chez les sujets diabétiques.

13. Utilisation selon la revendication 12, **caractérisée en ce que** l'agent antioxydant est notamment l'alpha-tocophérol, la Vitamine E et les sels de tocophéryl, le vanadium et les sels de vanadium, le coenzyme Q10 et ses dérivés, particulièrement l'ubiquinol 10, la Vitamine C et les sels d'acide ascorbique, le pyruvate, l'acide nicotinique, la Vitamine A, le rétinol, l'alpha-carotène, le beta-carotène, le lycopène, la lutéine, la zéaxanthine et autres caroténoïdes, la thiamine, la riboflavine, la pyridoxine et les Vitamines B1, B2 et B6, la mélatonine, les flavonoïdes, l'acide lipoïque, les acides aminés soufrés tels que la méthionine, la cystéine et la N-acetylcystéine et la taurine.

14. Utilisation selon la revendication 12 ou 13, **caractérisée en ce que** le vecteur d'ions fluorures est notamment le fluorure d'amine, le fluorure d'ammonium, les fluorures de métaux alcalins, notamment de sodium et de potassium, les fluorures alcalino-terreux, par exemple de calcium, les fluorures de titane, de zinc, d'étain, d'aluminium, ainsi que les dérivés de ces fluorures ; les fluorophosphates d'aluminium, les fluorophosphates de métaux alcalins, notamment de sodium et de potassium, par exemple le monofluorophosphate de sodium, les fluorophosphates alcalino-terreux, notamment de calcium, les fluorophosphates d'aluminium, par exemple le monofluorophosphate d'aluminium le difluorophosphate d'aluminium ; le fluorozirconate de sodium ; les fluorosilicates, tels que les fluorosilicates de sodium comme l'hexafluorosilicate de sodium.

15. Utilisation selon l'une quelconque des revendications 12 à 14, **caractérisée en ce qu'**elle comprend à titre de principes actifs un premier et un second agents antioxydants et au moins un vecteur d'ions fluorures.

16. Utilisation selon la revendication 15, **caractérisée en ce que** le second agent antioxydant est un produit sélénié, notamment un sélénate, par exemple le sélénate de sodium; un sélénite, par exemple le sélénite de sodium; un composé séléno-organique, par exemple un composé avec un acide aminé tel que la sélénométhionine, la sélénocystéine.

## Claims

1. A dental hygiene composition to fight and/or prevent periodontal pathologies in diabetic subjects, containing the active ingredients Vitamin E and at least one fluoride ion vector with a content of fluoride ions higher than 0.0001% in weight, preferably between 0.001 and 2% in weight in comparison with the total weight of the composition.

2. A composition according to claim 1, **characterized in that** Vitamin E is present in the form of salt, for example acetate.

3. A composition according to claim 1 or 2, **characterized in that** the fluoride ion vector is, in particular, the amine fluoride, ammonium fluoride, alkaline metal fluorides (for example sodium and potassium), earth-alkaline fluorides (for example, calcium), titanium, zinc, tin, aluminum fluorides, as well as derivatives of these fluorides ; aluminum fluorophosphates, alkaline metal fluorophosphates, in particular, sodium and potassium (for example, sodium monofluorophosphate), earth-alkaline fluorophosphates (for example calcium), aluminum fluorophosphates, in particular aluminum monofluorophosphate, aluminum difluorophosphate, sodium fluozirconate, fluorosilicates, such as sodium fluorosilicates such as sodium hexafluorosilicate.

4. A composition according to claim 3, **characterized in that** the fluoride ion vector is sodium fluoride, sodium monofluorophosphate or a mixture thereof.

5. A composition according to any of claims 1 to 4, **characterized in that** it comprises a second antioxidant agent in the active ingredients.

6. A composition according to claim 5, **characterized in that** the second antioxidant agent is a seleniated product.

7. A composition according to claim 6, **characterized in that** the seleniated product is, in particular, a selenate (for example, sodium selenate); a selenite (for example, sodium selenite) ; a selenoorganic compound (for example, a compound with an amino acid such as selenomethionine, selenocysteine).

8. A composition according to any of claims 1 to 7, **characterized in that** the total content of antioxidant agents is higher than 0.0001% in weight, preferably between 0.0005 and 2% in weight in comparison with the total weight of the said composition.

9. A composition according to any of claims 1 to 8, usable in the form of toothpaste, **characterized in that** it comprises the following compounds, expressed as a percentage of weight in comparison with the total weight of the composition:
- sodium fluoride 0.33
- sodium monofluorophosphate 0.76
- DL-alpha-tocopherol (Vitamin E) 0.5
- silica 21.00
- sorbitol 25.00
- sodium benzoate 4.00
- phosphates 0.3
- carraghenate 1.00
- titanium dioxide 1.00
- surface-active 2.00
- preservative 0.1
- aroma 0.1
- purified water to balance to

10. A composition according to any of claims 1 to 8, usable in the form of toothpaste, **characterized in that** it comprises the following compounds, expressed as a percentage of weight in comparison with the total weight of the composition:
- sodium fluoride 0.55
- DL-alpha-tocopherol (Vitamin E) 0.5
- silica 20.00
- glycerol 32.00
- carboxymethylcellulose 1.00
- titanium dioxide 1.00
- surface-active 1.00
- preservative 0.30
- aroma 0.2
- purified water to balance to

11. A composition according to any of claims 1 to 10, **characterized in that** it is formulated in the form of toothpaste, mouth spray, chewing gum, lozenge, mouth patch, mouthwash.

12. A usage of at least one antioxidant agent and at least one fluoride ion vector for the preparation of a dental hygiene composition to fight and/or prevent periodontal pathologies in diabetic subjects.

13. A usage according to claim 12, **characterized in that** the antioxidant agent is, in particular, the alpha-tocopherol, vitamin E and tocopherol salts, vanadium and vanadium salts, Q10 coenzyme and its derivatives, particularly ubiquinol 10, vitamin C and ascorbic acid salts, pyruvate, nicotinic acid, vitamin A, retinol, alpha-carotene, beta-carotene, lycopene, lutein, zeaxanthin and other carotenoids, thiamine, riboflavin, pyridoxine and vitamins B1, B2, and B6, melatonin, flavonoids, lipoic acid, sulfuric amino acids such as methionine, cysteine and N-acetylcysteine and taurine.

14. A usage according to claim 12 or 13, **characterized in that** the fluoride ion vector is, in particular, the amino fluoride, ammonium fluoride, alkaline metal fluorides, in particular sodium and potassium, earth-alkaline fluorides (for example, calcium), titanium, zinc, tin, aluminum fluorides, as well as derivatives of these fluorides ; the aluminum fluorophosphates, alkaline metal fluorophosphates, in particular, sodium and potassium (for example, sodium monofluorophosphate), earth-alkaline fluorophosphates, in particular, calcium, aluminum fluorophosphates (for example, aluminum monofluorophosphate) ; aluminum difluorophosphate ; sodium fluorozirconate ; fluorosilicates, such as sodium fluorosilicates such as sodium hexafluorosilicate.

15. A usage according to any of claims 12 to 14, **characterized in that** it comprises a first and second antioxidant agent in the active ingredients and at least one fluoride ion vector.

16. A usage according to claim 15, **characterized in that** the second antioxidant agent is a seleniated product, in particular, a selenate (for example, sodium selenate) ; a selenite (for example, sodium selenite) ; a selenoorganic compound (for example, a compound with an amino acid such as selenomethionine, selenocysteine).

## Patentansprüche

1. Zahnhygienezusammensetzung zum Bekämpfen und/oder Verhindern von Periodontalerkrankungen bei zuckerkranken Personen, die als Wirkstoffe Vitamin E und mindestens einen Fluoridionenvektor mit einem Fluoridionengehalt von über 0,0001 Gew.-%, vorzugsweise zwischen 0,001 und 2 Gew.-%, bezogen auf das Gesamtgewicht der Zusammensetzung, enthält.

2. Zusammensetzung nach Anspruch 1, **dadurch gekennzeichnet, dass** Vitamin E in der Form von Salz, zum Beispiel Acetat, vorhanden ist.

3. Zusammensetzung nach Anspruch 1 oder 2, **dadurch gekennzeichnet, dass** der Fluoridionenvektor insbesondere das Aminfluorid, Ammoniumfluorid, Alkalimetallfluoride (zum Beispiel von Natrium und Kalium), Erdalkalifluoride (zum Beispiel von Calcium), Titanfluoride-, Zink-, Zinn-, Aluminium- sowie Derivate dieser Fluoride, Aluminiumfluorphosphate, Alkalimetallfluorphosphate, insbesondere von Natrium und Kalium (zum Beispiel Natriummonofluorphosphat), Erdalkalifluorphosphate (zum Beispiel von Calcium), Aluminiumfluorphosphate, insbesondere Aluminiummonofluorphosphat, Aluminiumdifluorphosphat, Natriumfluorzirconat, Fluorsilicate, wie Natriumfluorsilicate, wie Natriumhexafluorsilicat, ist.

4. Zusammensetzung nach Anspruch 3, **dadurch gekennzeichnet, dass** der Fluoridionenvektor Natriumfluorid, Natriummonofluorphosphat oder eine Mischung davon ist.

5. Zusammensetzung nach einem der Ansprüche 1 bis 4, **dadurch gekennzeichnet, dass** sie ein zweites Antioxidationsmittel in den Wirkstoffen umfasst.

6. Zusammensetzung nach Anspruch 5, **dadurch gekennzeichnet, dass** das zweite Antioxidationsmittel ein seleniertes Produkt ist.

7. Zusammensetzung nach Anspruch 6, **dadurch gekennzeichnet, dass** das selenierte Produkt insbesondere ein Selenat (zum Beispiel Natriumselenat); ein Selenit (zum Beispiel Natriumselenit); eine selenorganische Verbindung (zum Beispiel eine Verbindung mit einer Aminosäure, wie Selenomethionin, Selenocystein) ist.

8. Zusammensetzung nach einem der Ansprüche 1 bis 7, **dadurch gekennzeichnet, dass** der Gesamtgehalt an Antioxidationsmitteln, bezogen auf das Gesamtgewicht der Zusammensetzung, höher als 0,0001 Gew.-% ist und vorzugsweise zwischen 0,0005 und 2 Gew.-% liegt.

9. Zusammensetzung nach einem der Ansprüche 1 bis 8, verwendbar in der Form von Zahnpasta, **dadurch gekennzeichnet, dass** sie die folgenden Verbindungen umfasst, ausgedrückt als Gewichtsprozentsatz bezogen auf das Gesamtgewicht der Zusammensetzung:
- Natriumfluorid 0,33
- Natriummonofluorphosphat 0,76
- DL-alpha-Tocopherol (Vitamin E) 0,5
- Silica 21,00
- Sorbit 25,00
- Natriumbenzoat 4,00
- Phosphate 0,3
- Carraghenat 1,00
- Titandioxid 1,00
- Tensid 2,00
- Konservierungsstoff 0,1
- Aroma 1,0
- gereinigtes Wasser Rest

10. Zusammensetzung nach einem der Ansprüche 1 bis 8, verwendbar in der Form von Zahnpasta, **dadurch gekennzeichnet, dass** sie die folgenden Verbindungen umfasst, ausgedrückt als Gewichtsprozentsatz bezogen auf das Gesamtgewicht der Zusammensetzung:
- Natriumfluorid 0,55
- DL-alpha-Tocopherol (Vitamin E) 0,5
- Silica 20,00
- Glycerol 32,00
- Carboxymethylcellulose 1,00
- Titandioxid 1,00
- Tensid 1,00
- Konservierungsstoff 0,30
- Aroma 0,2
- gereinigtes Wasser Rest

11. Zusammensetzung nach einem der Ansprüche 1 bis 10, **dadurch gekennzeichnet, dass** sie in der Form von Zahnpasta, Mundspray, Kaugummi, Pastille, Mundpflaster, Mundspülung formuliert ist.

12. Verwendung von mindestens einem Antioxidationsmittel und mindestens einem Fluoridionenvektor zur Herstellung einer Zahnhygienezusammensetzung zum Bekämpfen und/oder Verhindern von Periodontalerkrankungen bei zuckerkranken Personen.

13. Verwendung nach Anspruch 12, **dadurch gekennzeichnet, dass** das Antioxidationsmittel insbesondere das alpha-Tocopherol, Vitamin E und Tocopherolsalze, Vanadium und Vanadiumsalze, Q10-Coenzym und dessen Derivate, besonders Ubichinol 10, Vitamin C und Ascorbinsäuresalze, Pyruvat, Nicotinsäure, Vitamin A, Retinol, alpha-Carotin, beta-Carotin, Lycopin, Lutein, Zeaxanthin und andere Carotinoide, Thiamin, Riboflavin, Pyridoxin und Vitamine B1, B2 und B6, Melatonin, Flavonoide, Liponsäure, Schwefelaminosäuren wie Methionin, Cystein und N-Acetylcystein und Taurin ist.

14. Verwendung nach Anspruch 12 oder 13, **dadurch gekennzeichnet, dass** der Fluoridionenvektor insbesondere das Aminfluorid, Ammoniumfluorid, Alkalimetallfluoride, zum Beispiel von Natrium und Kalium, Erdalkalifluoride (zum Beispiel von Calcium), Titan-, Zink-, Zinn-, Aluminiumfluoride sowie Derivate dieser Fluoride, die Aluminiumfluorphosphate, Alkalimetallfluorphosphate, insbesondere von Natrium und Kalium (zum Beispiel Natriummonofluorphosphat), Erdalkalifluorphosphate, insbesondere von Calcium, Aluminiumfluorphosphate (zum Beispiel Aluminiummonofluorphosphat), das Aluminiumdifluorphosphat, das Natriumfluorzirconat, die Fluorsilicate, wie Natriumfluorsilicate, wie Natriumhexafluorsilicat, ist.

15. Verwendung nach einem der Ansprüche 12 bis 14, **dadurch gekennzeichnet, dass** sie ein erstes und ein zweites Antioxidationsmittel als Wirkstoffe und mindestens einen Fluoridionenvektor umfasst.

16. Verwendung nach Anspruch 15, **dadurch gekennzeichnet, dass** das zweite Antioxidationsmittel ein seleniertes Produkt ist, insbesondere ein Selenat (zum Beispiel Natriumselenat); ein Selenit (zum Beispiel Natriumselenit); eine organische Selenverbindung (zum Beispiel eine Verbindung mit einer Aminosäure, wie Selenomethionin, Selenocystein) ist.
